# EUROPÄISCHE PATENTSCHRIFT

(11) **EP 0 224 800 B1**
(45) Veröffentlichungstag und Bekanntmachung des Hinweises auf die Patenterteilung: **03.02.1993**
(21) Anmeldenummer: 86116036.4
(22) Anmeldetag: 20.11.1986
(51) Int. Cl.: C12M 3/00

(54) **Verfahren und Vorrichtung zum Kultivieren von Zellen**
Method and apparatus for cell culture
Méthode et appareil pour la culture de cellules

(30) Priorität: 26.11.1985 DE 3541738
(43) Veröffentlichungstag der Anmeldung: 10.06.1987
(73) Patentinhaber: BOEHRINGER MANNHEIM GMBH, 68298 Mannheim (DE)
(72) Erfinder: Kearns, Michael, Dr., D-5124 Seeshaupt (DE); Comer, Michael James, Dr. rer. nat., D-8139 Bernried (DE); Steegmans, Ulrich, D-8132 Tutzing (DE); Jungfer, Herbert, Dr. med., D-8132 Tutzing (DE)

(56) Entgegenhaltungen:
- EP-A- 0 039 055
- EP-A- 0 112 155
- FR-A- 2 238 759
- PATENT ABSTRACTS OF JAPAN, Band 10, Nr. 67 (C-333)[2124], 15. März 1986; & JP-A-60 207 581 (TEIJIN K.K.) 19-10-1985
- M.W. Glacken et al., Trends in Biotechnology, 1(4), 1983, pp. 102-108; G.B. Gori, Appl. Microbiology, 13(1), 1965, pp. 93-98

## Beschreibung

Die Erfindung bezieht sich auf ein Verfahren und eine Vorrichtung zum Kultivieren von Zellen im Rahmen der Biotechnologie. Insbesondere richtet sie sich auf die Vermehrung von Säugetierzellen in vitro.

Im Rahmen der Biotechnologie werden biologische Produkte, beispielsweise monoklonale Antikörper, Interferone, Impfstoffe, Plasminogen-Aktivatoren uva., mit Hilfe von Zellen, insbesondere Säugetierzellen, erzeugt. Die Wirtschaftlichkeit derartiger Verfahren ist wesentlich davon abhängig, daß sich die Zellen möglichst stark vermehren und in ihrem Innern eine möglichst hohe Konzentration der gewünschten Substanz erreicht wird. Um diese Ziele zu erreichen, werden an die Verfahren und Vorrichtungen zur großtechnischen Kultivierung von Zellen besondere Anforderungen gestellt. So muß insbesondere für eine ausreichende Nährstoffzufuhr sowie Zufuhr von Gasen, insbesondere Sauerstoff, Sorge getragen werden. Abfallprodukte, d.h. Stoffwechselprodukte der Zellen, die nicht in der Zellkultur gebraucht werden und deren Wachstum vielfach hemmen, müssen entfernt werden. Auch alle übrigen Umweltbedingungen, wie beispielsweise Temperatur und pH-Wert, sollen möglichst optimal sein.

In dem Artikel "Mammalian cell culture: engineering principles and scale-up" von M.W. Glacken et al in der Zeitschrift "Trends in Biotechnology", 1983, p. 102 - 108 sind diese Probleme und eine Vielzahl von Konstruktionsprinzipien für entsprechende Geräte zur Kultivierung von Zellen dargestellt.

Wie diesem Artikel zu entnehmen ist, muß man zwischen Suspensionszellen und trägerabhängigen Zellen unterscheiden. Während die Suspensionszellen frei in dem Zellkulturmedium schweben und wachsen können, bedürfen die trägerabhängigen Zellen, die auch als adhärente Zelle bezeichnet werden, eines festen Trägers, um überleben und wachsen zu können.

In dem Artikel wird neben den oben erwähnten Problemen besonderes die Problematik des "upscaling" herausgestellt. Es hat sich nämlich gezeigt, daß Methoden, die im Labormaßstab ausreichend funktionieren, für die Massenproduktion ungeeignet sind.

Traditionell werden Säugetierzellen ebenso wie Bakterienzellen primär als Suspensionskulturen in Bioreaktoren kultiviert, die auch als Fermenter bezeichnet werden. In einem derartigen Gefäß können die Umweltbedingungen genau kontrolliert werden. Eine Rühreinrichtung bewegt das Kulturmedium im Innern des Reaktors und sorgt somit für eine homogene Verteilung der Zellen. Eine entsprechende Kulturtechnik wird für adhärente Zellen dadurch möglich, daß sich diese auf kleinen Trägerkügelchen befinden, die als "Micro Carrier" bezeichnet werden. Diese schweben in dem Kulturmedium.

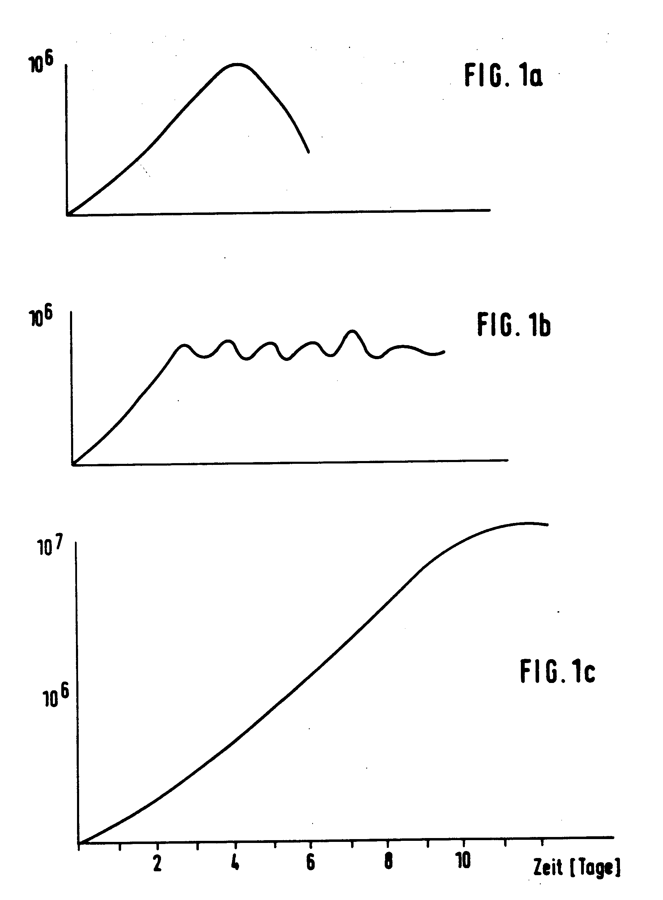

Die Zufuhr von Nährstoffen zu den Zellen und die Abfuhr von Abfallstoffen geschieht bei Suspensionskulturen in Bioreaktoren nach einem der folgenden drei Verfahren:
Im Batchbetrieb wird der Reaktor diskontinuierlich betrieben. Bei Beginn eines Ansatzes enthält das Kulturmedium üblicherweise ein Serum, beispielsweise fötales Kälberserum (FKS) und die notwendigen Nährstoffe, beispielsweise Glucose, Vitamine, Aminosäuren und Mineralien. Im Betrieb werden diese verbraucht, so daß das Medium immer mehr an Nährstoffen verarmt. Gleichzeitig nimmt die Konzentration von Abfallprodukten zu, was schließlich zu einer Behinderung des Zellwachstums führt. Die Folge davon ist ein Verlauf der Zelldichtekurve, wie er in Figur 1 a dargestellt ist. Die Zelldichte erreicht einen Maximalwert von etwa 10⁶ Zellen/ml und nimmt danach wieder ab. Deswegen wird die Kultivierung abgebrochen, wenn die maximale Zelldichte erreicht ist. Der Reaktorinhalt wird dann der Weiterverarbeitung zugeführt. Dieses Verfahren ist insoweit unbefriedigend, als sich die Umwelt der Zellen ständig ändert und deswegen während der meisten Zeit des Fermentationsvorgangs keineswegs optimal ist. Dies könnte zwar verbessert werden, indem das Kulturmedium mehrfach aufgefrischt wird, ohne dabei Zellen zu entnehmen. Dazu müßte jedoch wiederholt eine Teilmenge des Kulturmediums entfernt werden, obwohl es noch keineswegs verbraucht ist. Ein derartiges Verfahren ist äußerst aufwendig, weil die bekannten Kulturmedien schwer zu beschaffen und deswegen teuer sind.

In dieser Hinsicht besser ist das sogenannte "Fedbatch-Verfahren", bei dem während des Fermentationsvorganges nicht frisches Kulturmedium in seiner Gesamtheit, sondern nur die verbrauchten Nährstoffe kontinuierlich zugeführt werden. In der Praxis bringt dieses Verfahren jedoch keine wesentlichen Vorteile, weil die Zunahme der Abfallstoffe zu einem ähnlichen charakteristischen Verlauf der Zelldichte während des Kultivierungsvorganges führt wie beim reinen Batchverfahren.

Das dritte Verfahren ist das kontinuierliche Verfahren in einem sogenannten Chemostat oder Cytostat. Hier können die Umweltbedingungen gleichmäßig eingestellt werden, so daß die Zellen optimal wachsen können. Das Verfahren ist jedoch sehr aufwendig, weil kontinuierlich Kulturmedium zugeführt und abgeführt wird. Außerdem wird auch bei diesem Verfahren keine wesentlich höhere Zelldichte als bei den vorgenannten Verfahren erreicht, weil mit dem auslaufenden Zellkulturmedium ständig auch Zellen aus dem Reaktor abgeführt werden.

Die Zufuhr des Kulturmediums erfolgt bei den Chemostaten durch ein poröses Filterbauteil, beispielsweise einen Filter aus gesintertem Glas. Um die dabei auftretenden Probleme durch Verstopfung des Filters und unerwünschtes Zellwachstum auf seiner Oberfläche zu eliminieren, wurde wissenschaftlich diskutiert, die in der Bakteriologie bereits 1896 benutzte Dialysetechnik einzusetzen. In dem Artikel G.B. Gori "Chemostatic Concentrated Cultures of Heteroploid Mammalian Cell Suspensions in Dialyzing Fermentors", Appl. Microbiol. 1965, 93 bis 98, ist ein chemostatisches Verfahren beschrieben, bei dem in einem 2-Liter-Laborfermenter eine schlauchförmige Membran aus regenerierter Zellulose angeordnet ist. Durch diesen Schlauch zirkuliert unter leichtem Überdruck von etwa 1.000 Pa ein Kulturmedium, welches 10 % Kälberserum enthält.

Im Zusammenhang mit der Fermentation von Mikroorganismen, die zur Käseherstellung benötigt werden, wird in der FR-A-2 238 759 die Verwendung eines Dialysators beschrieben, der außerhalb des Fermenters in einem externen Kreislauf von einer die Mikroorganismen enthaltenden Kulturlösung durchströmt wird. Zur Separierung der relativ großen Mikroorganismen kann dabei eine verhältnismäßig grobporige Membran mit einer Porengröße von 0,2 µ eingesetzt werden. Dennoch wird eine relativ große Membran-Austauschfläche von mindestens etwa 1,2 m²/l Kulturflüssigkeit für erforderlich gehalten. Es ist daher nicht erstaunlich, daß dieses Verfahren, welches mehr als zehn Jahre vor der vorliegenden Erfindung publiziert wurde, im Hinblick auf die Kultivierung von Säugetierzellen oder Hypridomen in der Fachwelt nicht einmal diskutiert wurde.

Unabhängig davon, welches der drei für Suspensionskulturen beschriebenen Betriebsverfahren gewählt wird, sind also die Ergebnisse nicht befriedigend, insbesondere weil der Verbrauch an wertvollem Zellkulturmedium zu hoch ist und weil eine höhere maximale Zelldichte wünschenswert ist. Beispielsweise beträgt die erreichte Zelldichte bei dem in dem Artikel von Gori beschriebenen Dialyse-Chemostat-Verfahren 1,2 x 10⁶ Zellen/ml. Das Volumen des zirkulierenden serumhaltigen Kulturmediums ist dabei das Zehnfache des Fermentervolumens, so daß eine sehr große Menge Serum benötigt wird. Die Zelldichte ist von besonderer Bedeutung, weil die vorliegenden Erfahrungen zeigen, daß die Konzentration der gewünschten Produkte in den Zellen mit zunehmender Zelldichte zunimmt. Eine Steigerung der Zelldichte führt deshalb zu einer überproportionalen Steigerung der Ausbeute an Produkt.

Um eine weitere Verbesserung zu erreichen , wurden neue Verfahren vorgeschlagen.

Die sogenannten "Systeme mit künstlichen Kapillaren" bestehen im wesentlichen aus einem Bündel von Dialyse-Hohlfasern, die im Inneren eines hohlen Zylinders angeordnet sind. Sie werden vor allen Dingen für die Kultivation adhärenter Zellen empfohlen. Dabei werden die Zellen auf der Außenseite der Hohlfasern angelagert , befinden sich als in den Zwischenräumen zwischen den Hohlfasern innerhalb des zylindrischen Gehäuses. Ein Kulturmedium strömt durch die Innenseite der Kapillaren, Luft wird durch die Gehäuseaußenseite den Zellen zugeführt. Mit einem derartigen Verfahren wird eine verhältnismäßig gleichmäßige und sparsame Zufuhr der Nährstoffe zu den Zellen erreicht. Auch die Zelldichte konnte geringfügig erhöht werden. Obwohl die Zufuhr der Nährstoffe gleichmäßig ist, zeigt sich jedoch, daß die Zellen unterschiedlich gut versorgt werden. Dies wird insbesondere darauf zurückgeführt, daß sie in mehreren Schichten auf den Kapillaren sitzen. Dadurch wird auch die Sauerstoffzufuhr beschränkt, was zu einer erhöhten Produktion an Abfallprodukten führen kann.

Derartige Hohlfaserfermenter sind in den europäischen Patentanmeldungen mit den Publikationsnummer 112 154 und 112 155 beschrieben. In der letztgenannten Druckschrift ist das System insoweit variiert, als die Zellen mittels einer Pumpe im Kreislauf durch den Kultivationsmodul gepumpt werden.

Hohlfasern, deren Wände aus einem Membranmaterial bestehen, werden auch bei dem in der japanischen Offenlegungsschrift 60-20 75 81 beschriebenen Zellkultivierungsverfahren eingesetzt. Die Hohlfasern befinden sich im Kulturmedium im Innenraum eines Fermenters. Das Innere der Hohlfasern ist über eine einzige Leitung, die sowohl der Zufuhr als auch der Abfuhr von Flüssigkeiten dient mit dem Außenraum verbunden. Mit Hilfe einer Pumpe werden durch diese Leitung Abfallstoffe der Zellen durch die Wandung der Hohlfasern hindurch abgesaugt bzw. in einem zeitlich davon getrennten Zyklus sterilisiertes Wasser, welches den Zellen nicht schadet, zugeführt. Diesem sterilisierten Wasser können gegebenenfalls auch Nährstoffe für die Zellen beigefügt sein. Im Betrieb wird abwechselnd in die Kulturflüssigkeit hineingepumpt und aus dieser abgesaugt. Dieser Zyklus wird fortlaufend wiederholt. Um die Nachteile der diskontinuierlichen Betriebsweise zu vermeiden werden bevorzugt eine geradzahlige Anzahl von Hohlfaserbündeln eingesetzt, welche alternierend betrieben werden, d. h. während durch die eine Hälfte der Hohlfaserbündel aus dem Kulturmedium abgesaugt wird, wird durch die andere Hälfte der Hohlfaserbündel Flüssigkeit zugeführt. Dieses Verfahren ist verhältnismäßg aufwendig und bringt die Gefahr mit sich, daß die Hohlfasern durch die ständige alternierende Druckbelastung von der einen und von der anderen Seite beschädigt werden.

Eine andere neuartige Technologie sieht vor, die Zellen in kugelförmige Mikrokörper aus einem semipermeablen Membranmaterial einzuschließen. Dies wird in dem zitierten Artikel als einziges neues Verfahren für die Kultivierung von Suspensionszellen bezeichnet. Es führt zu einer Erhöhung der Zelldichte in den kleinen Kugelkörpern, was wiederum eine Erhöhung der Produktausbeute nach sich zieht. Das Ernten der Zellen ist einfach, weil sich die Kugelkörperchen aufgrund der Schwerkraft absetzen, während Einzelzellen in der Regel zentrifugiert werden müssen. Diesen Vorteilen stehen sehr hohe Kosten für das Einkapseln der Zellen gegenüber. Außerdem ist die Sauerstoffzufuhr zu den Zellen beschränkt, was wiederum den Kultivationserfolg mindert.

Vor diesem Hintergrund stellt sich die vorliegende Erfindung die Aufgabe, ein einfaches und kostengünstiges Verfahren und eine entsprechende Vorrichtung zur Kultivierung von Zellen zur Verfügung zu stellen, die eine erhöhte Zelldichte und infolgedessen eine erhöhte Produktausbeute ermöglichen.

Diese Aufgabe wird durch die in den Ansprüchen gekennzeichnete Erfindung gelöst.

Die Erfindung geht von dem klassischen Fermenter aus, in dessen Innenraum sich die Zellen befinden. Sie eignet sich besonders für Suspensionszellen, ist jedoch auch auf adhärente Zellen anwendbar, insbesondere wenn diese auf Mikro-Carriern angesiedelt sind, wie dies in dem zitierten Artikel beschrieben ist. In dem Reaktor werden die Zellen mit Hilfe einer Rühreinrichtung in weitgehend homogener Suspension gehalten. Die Umweltbedingungen, also insbesondere die Temperatur, der Sauerstoffpartialdruck und der pH-Wert werden mit den für Bioreaktoren bekannten Verfahren kontrolliert und im optimalen Bereich konstant gehalten.

Durch die Membranhohlfasern werden die Nährstoffe zu den Zellen zugeführt und die Abfallprodukte von den Zellen abgeführt. Dies geschieht mit Hilfe einer semipermeablen Membran, die zwei Räume voneinander trennt. Auf der Außenseite der Membran befindet sich das Kulturmedium in dem Innenraum des Reaktors. Es enthält neben den Zellen insbesondere die in den bekannten Kulturmedien stets enthaltenen Eiweißsubstanzen, insbesondere das oben erwähnte fötale Kälberserum. Von dem Kulturmedium getrennt ist ein Nährmedium, das lediglich die relativ niedermolekularen Nährstoffe enthält, die von den Zellen laufend verbraucht werden.

Eine semipermeable Membran ist eine Trennwand, durch die manche Moleküle hindurchdiffundieren können, während andere Moleküle zurückgehalten werden. Für die vorliegende Erfindung muß die Durchlässigkeit der Membran so ausgelegt sein, daß sie für die relativ niedermolekularen Nährstoffe der Zellen sowie für deren Abfallprodukte durchlässig, für die höhermolekularen Bestandteile des Kulturmediums aber undurchlässig ist. Die Membran hält infolgedessen die hochmolekularen und besonders wertvollen Bestandteile des Kulturmediums in diesem fest. Sie werden während des Kultivationsvorgangs praktisch nicht verbraucht, so daß sie nicht zugeführt werden müssen. Die niedermolekularen Nährstoffe dagegen können die Membran passieren, so daß auf beiden Membranseiten näherungsweise die gleiche Konzentration an diesen Stoffen vorherrscht. Ähnliches gilt auch für die Abfallprodukte der Zellen, die aus dem Kulturmedium durch die Membran in das Nährmedium gelangen.

Beide Medien werden nun derartig in Bewegung gehalten, daß sie auf der jeweiligen Membranseite fortlaufend vorbeiströmen. Dabei strömt das Nährmedium im Innern der Hohlfasern, während das Kulturmedium die Außenseite der Hohlfasern umspült. Dadurch wird einerseits auf der Nährstoffmedium-Seite der Membran ständig eine ausreichend hohe Nährstoffkonzentration und eine ausreichend niedrige Abfallstoffkonzentration aufrechterhalten. Andererseits ist auch das Kulturmedium insgesamt so weitgehend homogen, daß überall weitgehend optimale Wachstumsbedingungen für die Zellen herrschen. Im einzelnen muß die Konzentration der Nährstoffe in dem Nährmedium, die Strömungsgeschwindigkeit, mit der das Nährmedium an der Dialysemembran vorbeigeführt wird und die Austauschfläche der Dialysemembran sowie die Strömungsgeschwindigkeit, mit der das Kulturmedium an der Membran vorbeiströmt, so aufeinander abgestimmt sein, daß im stationären Zustand die Konzentration der Nährstoffe in dem Kulturmedium optimiert ist und daß die Konzentration der Abfallstoffe in dem Kulturmedium so gering ist, daß das Wachstum der Zellen nicht wesentlich gestört wird.

Überraschenderweise hat sich gezeigt, daß auf diese Weise mit Hilfe einer Dialysemembran nicht nur deren unmittelbare Umgebung in einem Dialysator, sondern der gesamte Innenraum eines Bioreaktors außerordentlich gut mit Nährstoffen versorgt werden kann und daß die Abfallprodukte auf einer so geringen Konzentration gehalten werden können, daß sie das Wachstum kaum stören. Insgesamt ergeben sich damit, wie im folgenden noch näher dargelegt wird, Zelldichten und Produktausbeuten, die den klassischen Suspensionskulturen weit überlegen sind. Dennoch ist das Verfahren einfach und für die Herstellung großer Mengen sehr gut geeignet.

Das Bündel der Membranhohlfasern, das an beiden Enden mit entsprechenden Anschlußköpfen versehen ist, ist in dem Bioreaktor frei angeordnet. Das Kulturmedium wird dabei durch die in dem Bioreaktor vorhandene Rühreinrichtung an der semipermeablen Membran vorbeigeführt. Bevorzugt ist die Rühreinrichtung so abgestimmt, daß eine Umwälzung des Kulturmediums mit einem hohen Austausch des Mediums im Bereich der Membran erreicht wird.

Überraschenderweise ergibt sich bei diesem einfachen Verfahren ein ausreichend guter Austausch zwischen Nährmedium und Kulturmedium. Durch die offene Anordnung der Membran im Innern des Bioreaktors gibt es keine engen Kanäle, in denen es zu einer Verstopfung durch die Zellen oder die Eiweißbestandteile des Mediums kommen könnte.

Die Erfindung erzielt vor allem folgende Vorteile:
- Die Zelldichte ist annähernd 10 mal so groß wie bei den klassischen Suspensionskulturverfahren in Bioreaktoren. Sie wird in etwa acht Tagen erreicht und nähert sich asymptotisch einem Maximalwert (Figur 1 c), d.h. es kommt nicht zu einem Abfall der Zelldichte nach Erreichen des Maximums. Infolgedessen ist es nicht notwendig, den Kultivationsvorgang ständig zu überwachen, um den optimalen Zeitpunkt für die Ernte der Zellen festzulegen. Die Konzentrationen der Nährstoffe und der toxischen Abfallprodukte sind nahezu konstant und können sehr gut optimiert werden.
- Die Produktausbeuten sind - insbesondere wegen der hohen Zelldichten - bis zu 30 mal so hoch wie bei den klassischen Verfahren.
- Es wird sehr viel weniger Kulturmedium verbraucht. Infolgedessen wird in hohem Maße insbesondere Serum gespart. Auch dies hängt teilweise mit der hohen Zelldichte zusammen, weil die gleiche Kulturmediummenge bei dem erfindungsgemäßen Verfahren zur Produktion einer sehr viel größeren Menge an Zellen und Zellprodukten ausreicht. Hinzu kommt, daß, wie erwähnt, das Serum im Gegensatz zu den geschilderten Perfusionsverfahren während eines Ansatzes nicht erneuert oder ergänzt werden muß. Lediglich die wesentlich kostengünstigeren Nährstoffe werden verbraucht.
- Gegenüber den oben erwähnten neuen Verfahren (Zellzucht auf künstlichen Kapillaren, Einkapselung in Membrankapseln), die teilweise ebenfalls eine recht hohe Zelldichte erreichen, ist die Erfindung vor allem dadurch überlegen, daß in dem Bioreaktor die Umweltbedingungen sehr genau erfaßt und geregelt werden können. So läßt sich beispielsweise der pH-Wert und der Sauerstoffpartialdruck ständig messen und entsprechend korrigieren. Wegen der Homogenität der Suspension in dem Bioreaktor sind die Werte überall näherungsweise gleich. Außerdem ist das erfindungsgemäße Verfahren bestechend einfach. Die Investitionsaufwendungen sind verhältnismäßig gering, die Möglichkeiten des Up-scaling sind sehr gut und es müssen keine aufwendigen zusätzlichen Verfahrensschritte, wie beispielsweise beim Einkapseln ergriffen werden.

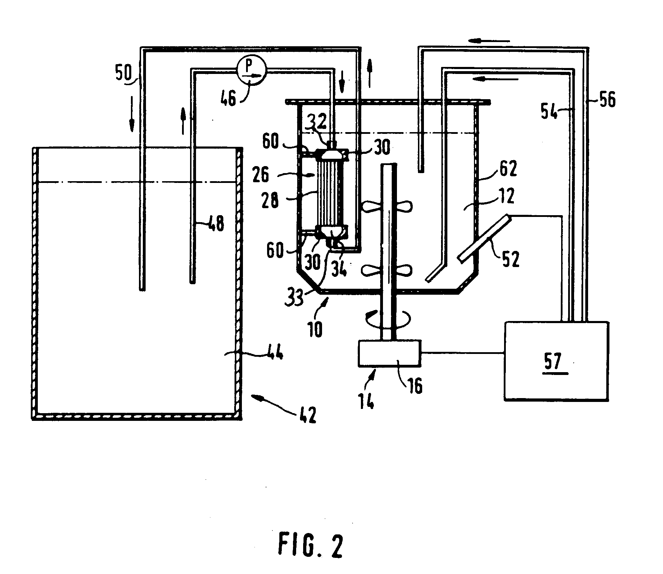

Die Erfindung und die mit ihr erzielbaren Vorteile werden im folgenden anhand von Ausführungsbeispielen, die in den Figuren 2 und 3 dargestellt sind, näher erläutert. Es zeigen:
Fig. 1 a bis Fig. 1 c die zeitliche Entwicklung der Zelldichte in verschiedenen Suspensionszellkulturen im Bioreaktor etwa im gleichen Maßstab, und zwar Fig. 1 a für einen einfachen Batch-Betrieb, Fig. 1 b für den Betrieb mit kontinuierlicher Zufuhr von Kulturmedium (Chemostat) und Fig. 1 c für das erfindungsgemäße Verfahren.
Fig. 2 eine schematische Darstellung einer erfindungsgemäßen Vorrichtung, bei der sich die Membran im Bioreaktor befindet.

Der in Fig. 2 dargestelle Bioreaktor 10 ist mit einem Kulturmedium 12 gefüllt. Dieses Kulturmedium enthält hochmolekulare Eiweißbestandteile, insbesondere Seren wie z.B. fötales Kälberserum. Zur Homogenisierung des Kulturmediums ist eine Rühreinrichtung 14 vorgesehen. Diese besteht im wesentlichen aus einem Motor 16, einer die Reaktorwand durchdringenden Achse 18 und an dieser befestigten Rührelementen 20. Die Rührelemente 20 sind bevorzugt schiffsschraubenartig gestaltet (sogenannte Schiffsschraubenimpeller). Eine derartige Form von Rührelementen, die sich durch abgerundete Begrenzungslinien und eine von außen nach innen kontinuierlich zunehmende Steigung des Anstellwinkels der Rührblätter auszeichnet, hat sich als besonders geeignet erwiesen, um den für die Erfindung notwendigen Grad der Homogenisierung des Kulturmediums zu erreichen, ohne die äußerst empfindlichen Zellen der Kultur zu beschädigen.

Membranhohlfasern 28 sind zwischen den beiden Anschlußköpfen 30 innerhalb des Bioreaktors 10 frei gespannt und bilden ein Dialysator 26, dessen Hohlfaserbündel nicht von einer Gehäusewandung umgeben ist. Die Anschlußköpfe 30 sind mittels einer geeigneten Halterung 60 an der Wand 62 des Bioreaktors 10 befestigt.

Die Anschlußköpfe sind so gestaltet, daß die offenen Enden der Hohlfasern mit den Anschlußstutzen 32 und 33 für das Dialysat in hydraulischer Verbindung stehen. Die Innenräume aller Hohlfasern und die Verbindungskanäle bis zu den Anschlußstutzen bilden also einen miteinander verbundenen ersten Raum 34, den man auch als Dialysatraum bezeichnen kann.

Der Hohlfaserdialysator ist ähnlich aufgebaut wie dies bei Dialysatoren für die Blutdialyse (sogenannte "künstliche Niere") üblich ist. Die Leitung 48, der Dialysatraum 34 und die Leitung 50 bilden einen geschlossenen Kreislauf für das Nährmedium 44. Ein Austausch zwischen Nährmedium 44 und dem Kulturmedium 12 in dem Bioreaktor 10 ist nur über die als Dialysemembran wirkende Wände der Hohlfasern 28 möglich.

Das Kulturmedium wird in dem Bioreaktor so umgewälzt, daß es im Bereich der Umgebung des Dialysators 26 ständig in Bewegung ist und an der Dialysemembran vorbeigeführt wird. Der zweite Raum des Dialysators ist in diesem Fall kein abgeschlossener Raum, sondern bildet einen nicht abgegrenzten Teil des Innenraums des Bioreaktors 10. Überraschenderweise führt ein derartig einfacher Aufbau zu einem ausgezeichneten Ergebnis. Dabei ist der Austausch der Nährstoffe und der Abfallstoffe über die Dialysemembran so gut, daß deren Konzentration in dem Bioreaktor weitgehend konstant im optimalen Bereich gehalten werden kann.

In einem Nährmediumtank 42 befindet sich Nährmedium 44. Dieses besteht aus einer geeigneten Trägerflüssigkeit und verschiedenen niedermolekularen Bestandteilen, die für die Ernährung der Zellen benötigt werden. Hierzu gehören Glucose, Vitamine, Aminosäuren und Mineralien. Mittels einer Pumpe 46 wird Nährmedium über die Leitung 48 dem Dialysatraum 34 des Dialysators 26 zugeführt. Dort durchströmt es die Hohlfasern 28 und fließt durch die Leitung 50 in den Nährmediumtank 42 zurück.

Das Nährmedium 44 in dem Nährmediumtank 42 wird ausreichend häufig erneuert oder ergänzt, daß die gewünschte Nährstoffkonzentration auf der Nährmediumseite der Dialysemembran aufrechterhalten bleibt. Dies kann entweder dadurch realisiert sein, daß der Nährmediumtank 42 so groß ist, daß die im Laufe eines Kultivationsvorganges sich einstellende Änderung der Nährstoffkonzentration nicht stört oder das Nährmedium kann während eines Kultivationsvorganges erneuert werden. Auch eine kontinuierliche Zufuhr von Nährstoffen, die die Konzentration in dem Nährmediumtank 42 aufrechterhält, kann vorteilhaft sein.

In jedem Fall ist wesentlich, daß der erste Raum des Dialysators für das Nährmedium und der zweite Raum des Dialysators für das Kulturmedium, die selbstverständlich vollkommen gegeneinander getrennt sein müssen, so daß ein Austausch nur über die Dialysemembran möglich ist, so gestaltet sind, daß die notwendige Austauschfläche sichergestellt ist. Bevorzugt liegt die Austauschfläche für das erfindungsgemäße Verfahren zwischen 0,01 m² je Liter Kulturmedium und 0,3 m² je Liter Kulturmedium, besonders bevorzugt zwischen 0,03 m² und 0,2 m² je Liter Kulturmedium. Außerdem muß der Strömungsverlauf insbesondere in dem vom Kulturmedium durchströmten zweiten Raum 36 so sein, daß die Zellen nirgends zu einer Verstopfung führen können und das Kulturmedium den Raum möglichst glattflächig durchströmen kann.

Die Dialysemembran kann aus einer Vielzahl für derartige Zwecke bekannter Materialen hergestellt sein. Bekannt sind insbesondere Zelluloseacetat, Acrylcopolymere und Polysulfonfasern. Besonders bevorzugt für die Erfindung sind Fasern aus Cupraammonium Rayon. Wesentlich ist, daß die Membran für die niedermolekularen Nährstoffe durchlässig, für die hochmolekularen Eiweißsubstanzen des Kulturmediums aber undurchlässig ist. Praktisch bewährt hat sich eine Membran mit einer Durchlaßgrenze (molecular cut-off) bei einem Molekulargewicht von etwa 10 000, jedoch kann auch eine Membran mit einer Durchlaßgrenze von 100 000 je nach Einsatzsfall zweckmäßig sein.

Der Bioreaktor ist mit Einrichtungen zur Überwachung und Konstanthaltung der Umweltbedingungen für die Zellen in dem Kulturmedium 12 versehen. Insbesondere sind Sensoren für den pH-Wert, den Sauerstoffpartialdruck und die Temperatur in dem Bioreaktor 10 vorhanden. Sie sind in der Zeichnung in ihrer Gesamtheit mit 52 bezeichnet. Zu den Einrichtungen zur Überwachung und Konstanthaltung der Umweltbedingungen gehören weiterhin eine Leitung zur Zufuhr von Gasen 54, durch die insbesondere Sauerstoff zugeführt wird und eine Leitung 56 zur Zufuhr von Säuren und/oder Laugen, mit deren Hilfe der pH-Wert kontrolliert wird. Diese Einrichtungen sind für Suspensionskulturen in Bioreaktoren allgemein bekannt. Die vorliegende Erfindung zeichnet sich jedoch besonders dadurch aus, daß sie nicht nur wie bei den bekannten Bioreaktoren eine sehr genaue Kontrolle der Temperatur, des Sauerstoffpartialdruckes und des pH-Werte ermöglicht, sondern daß gleichzeitig auch die Konzentrationen der Nährstoffe und der Abfallprodukte der Zellen gut kontrolliert und weitgehend konstant in einem Bereich gehalten werden können, der ein optimales Zellwachstum ermöglicht.

Die erfindungsgemäße Vorrichtung wird bevorzugt in folgender Weise betrieben:
Das Kulturmedium wird zusammen mit einer Ausgangskultur der Zellen in den Bioreaktor gefüllt und dieser wird verschlossen. Auch der Nährstofftank wird mit Nährmedium gefüllt und verschlossen. Danach werden die Pumpen und Rühreinrichtungen sowie die Einrichtungen zum Kontrollieren der Umweltbedingungen in Betrieb gesetzt, so daß der Kultivationsvorgang ablaufen kann. Während des Kultivationsvorganges muß die Anlage lediglich überwacht und dafür Sorge getragen werden, daß die Nährstoffkonzentration in der weiter oben beschriebenen Weise aufrechterhalten wird.

Während des Betriebs strömt an der Membran des Dialysators einerseits das Nährmedium, andererseits das Kulturmedium mit kontrollierter Geschwindigkeit vorbei. Die niedermolekularen Bestandteile beider Medien werden über die Dialysemembran ausgetauscht. Die Geschwindigkeit, mit der dabei einerseits Nährstoffe aus dem Nährmedium in das Kulturmedium und andererseits Abfallprodukte der Zellen aus dem Kulturmedium in das Nährmedium übertreten, ist bekanntermaßen von den Konzentrationen der entsprechenden Stoffe auf den beiden Seiten der Membran und der Membranfläche abhängig. Die Stoffkonzentrationen auf beiden Seiten hängen wiederum von den Ausgangskonzentrationen in dem Reservoiren, also einerseits in dem Bioreaktor und andererseits in dem Nährmediumvorrat sowie von der Geschwindigkeit ab, mit der die Medien an der Membran vorbeiströmen. Aufgrund dieser bekannten Zusammenhänge ist es jedem Fachmann möglich, die Einzelheiten so einzustellen, daß während der Kultivierung die Nährstoffkonzentration und die Abfallstoffkonzentration in dem Kulturmedium im Bioreaktor im optimalen Bereich liegen. Nach einer Anlaufphase stellt sich bei entsprechender Abstimmung ein stationärer Zustand ein, in dem sowohl die Nährstoffkonzentration als auch die Abfallkonzentration im wesentlichen konstant sind.

Da, wie erwähnt, auch die übrigen Umweltbedingungen im optimalen Bereich konstant gehalten werden, wächst die Zellkultur in der in Figur 1 c dargestellten Weise heran, bis die Zelldichte einen Maximalwert erreicht der weit höher ist als bei den bekannten Kultivierungsverfahren. Dieser Wert bleibt im wesentlichen konstant. Wenn er erreicht wird, kann der Bioreaktor geöffnet und die Zellkultur in bekannter Weise geerntet werden.

In Tabelle 1 werden die Ergebnisse, die sich mit dem erfindungsgemäßen Verfahren und einer entsprechenden Vorrichtung erzielen lassen, mit den Ergebnissen der Zellkultivierung in einer Bioreaktor-Suspensionskultur, die im Batch-Betrieb ohne Dialysator betrieben wurde, verglichen. Die Ergebnisse sind für vier verschiedene Hybridomazellinien angegeben. Man erkennt aus der Tabelle, daß je nach Zelltyp eine Verbesserung der maximalen Zelldichte zwischen etwa einem Faktor 6 und einem Faktor 12 erreicht wird. Die maximale Antikörperkonzentration in der Kultur wird noch erheblich stärker verbessert, nämlich um einen Faktor 12 bis zu einem Faktor von mehr als 30.

| Zellinie | Ursprung | maximale Zelldichte [Anzahl pro ml] | | Maximale Antikörperkonzentration [ug pro ml] | |
|---|---|---|---|---|---|
| | | bekannte (Batch) Bioreaktor-Kultur | erfindungsgem. Bioreaktor-Kultur | bekannte (Batch) Bioreaktor-Kultur | erfindungsgem. Bioreaktor-Kultur |
| 1. | Maus | 10⁶ | 6 x 10⁶ | 20 | 300 |
| 2. | Mensch | 1.5 x 10⁶ | 2 x 10⁷ | 0.3 | 11 |
| 3. | Mensch | 1.5 x 10⁶ | 10⁷ | 0.5 | 13 |
| 4. | Mensch | 1.5 x 10⁶ | 10⁷ | 0.8 | 10 |

## Patentansprüche

1. Verfahren zum Kultivieren von Zellen, bei dem eine Zellkultur in einem Reaktor unter kontrollierten Umweltbedingungen in einem Kulturmedium mittels einer Rühreinrichtung in weitgehend homogener Suspension gehalten wird, wobei
in dem Reaktor semipermeable Membranhohlfasern angeordnet sind,
die Membranhohlfasern eine Durchlaßgrenze haben, welche so bemessen ist, daß sie für die Nährstoffe und Abfallprodukte der Zellen durchlässig sind, für die höhermolekularen Eiweißbestandteile des Kulturmediums aber undurchlässig sind,
durch den Innenraum der Membranhohlfasern ein von dem Kulturmedium getrenntes Nährmedium in einem von dem Kulturmedium durch die Membranhohlfasern getrennten Strömungsweg strömt und
das Kulturmedium in dem Reaktorraum derartig umgewälzt wird, daß es auf der Außenseite der Membranhohlfasern vorbeigeführt wird,
so daß Nährstoffe aus dem Nährmedium durch die Wände der Membranhohlfasern in das Kulturmedium gelangen und Abfallstoffe aus dem Kulturmedium in das Nährmedium gelangen, höhermolekulare Eiweißbestandteile jedoch in dem Kulturmedium festgehalten werden.

2. Verfahren nach Anspruch 1, **dadurch gekennzeichnet,** daß die Austauschfläche der Membranhohlfasern mindestens 0,01 m² pro Liter Kulturmedium und höchstens 0,3 m² pro Liter Kulturmedium, bevorzugt mindestens 0,03 m² pro Liter Kulturmedium und höchstens 0,2 m² pro Liter Kulturmedium beträgt.

3. Vorrichtung zum Kultivieren von Zellen mit einem Reaktor (10), in welchem ein Kulturmedium (12) mittels einer Rühreinrichtung in weitgehend homogener Suspension gehalten wird, und der Einrichtungen (52, 54, 46, 57) zum Kontrollieren bestimmter Umweltbedingungen aufweist, wobei
in dem Reaktor Membranhohlfasern (28) vorgesehen sind, die einen Dialysatraum (34), der den Innenraum der Membranhohlfasern (28) einschließt, von dem Innenraum des Reaktors (10) abteilen,
die Durchlaßgrenze der Membranhohlfasern (28) so bemessen ist, daß sie für die Nährstoffe und die Abfallprodukte der Zellen durchlässig, für die höhermolekularen Eiweißbestandteile des Kulturmediums aber undurchlässig sind,
der Dialysatraum (34) mit einem Vorrat an Nährmedium (44) über eine Zuführungsleitung (48) und eine Rückführungsleitung (50) verbunden ist und Einrichtungen zum Zuführen des Nährmediums (44) von dem Vorrat an Nährmedium über die Zuführungsleitung (48) zu dem Dialysatraum (34) und zum Rückführen von dem Dialysatraum (34) über die Rückführungsleitung (50) zu dem Vorrat vorgesehen sind und
eine Umwälzeinrichtung vorgesehen ist, durch die das Kulturmedium (12) in dem Innenraum des Reaktors (10) derartig umgewälzt wird, daß es an den Membranhohlfasern (28) vorbeigeführt wird.

4. Vorrichtung nach Anspruch 3, bei welchem die Austauschfläche der Membranhohlfasern mindestens 0,01 m² pro Liter Kulturmedium und höchstens 0,3 m² pro Liter Kulturmedium, bevorzugt mindestens 0,03 m² pro Liter Kulturmedium und höchstens 0,2 m² pro Liter Kulturmedium, beträgt.

5. Vorrichtung nach einem der Ansprüche 3 oder 4, bei welcher die Umwälzeinrichtung mindestens einen Schiffsschraubenimpeller (20) einschließt.

## Claims

1. Process for the culturing of cells in which a cell culture in a reactor is kept in substantially homogeneous suspension under controlled environmental conditions in a culture medium by means of a stirring device, whereby semipermeable membrane hollow fibres are provided in the reactor, the membrane hollow fibres have a limit of permeability which is so rated that they are permeable for the nutrient materials and waste products of the cells but are impermeable for the high molecular protein components of the culture medium, through the inner space of the membrane hollow fibres flows a nutrient medium separate from the culture medium in a flow path separated from the culture medium by the membrane hollow fibres, the culture medium is cycled in the reactor chamber in such a manner that it is passed by the outer side of the membrane hollow fibres so that the nutrient materials pass from the nutrient medium through the walls of the membrane hollow fibres into the culture medium and waste materials pass from the culture medium into the nutrient medium but high molecular protein components are retained in the culture medium.

2. Process according to claim 1, characterised in that the exchange surface of the membrane hollow fibres amounts to at least 0.01 m² per litre of culture medium and at most 0.3 m² per litre of culture medium, preferably at least 0.03 m² per litre of culture medium and at most 0.2 m² per litre of culture medium.

3. Device for the culturing of cells with a reactor (10), in which a culture medium (12) is kept in substantially homogeneous suspension by means of a stirring device, and which has devices (52,54,46,57) for the controlling of definite environmental conditions, whereby, in the reactor, membrane hollow fibres (28) are provided which separate a dialysis chamber (34), which includes the inner spaces of the membrane hollow fibres (28), from the inner chamber of the reactor (10), the limit of permeability of the membrane hollow fibres (28) is so rated that they are permeable for the nutrient materials and the waste products of the cells but are impermeable for the high molecular protein components of the culture medium, the dialysate chamber (34) is connected with a reservoir of nutrient medium (44) via an introduction pipe (48) and a return pipe (50) and devices are provided for the introduction of the nutrient medium (44) from the reservoir of nutrient medium via the introduction pipe (48) to the dialysis chamber (34) and for the return from the dialysis chamber (34) via the return pipe (50) to the reservoir and a circulating device is provided by means of which the culture medium is circulated in the inner chamber of the reactor (10) in such a manner that it is passed by on the membrane hollow fibres (28).

4. Device according to claim 3, in which the exchange surface of the membrane hollow fibres amounts to at least 0.01 m² per litre of culture medium and at most 0.3 m² per litre of culture medium, preferably at least 0.03 m² per litre of culture medium and at most 0.2 m² per litre of culture medium.

5. Device according to one of claims 3 or 4, in which the circulating device includes at least one propelling screw impeller (20).

## Revendications

1. Procédé pour la culture de cellules, dans lequel une culture de cellules est maintenue en suspension extrêmement homogène dans un milieu de culture, à l'aide d'un agitateur dans un réacteur, dans des conditions d'environnement contrôlées, procédé dans lequel
une membrane de fibres creuses semiperméable est disposée dans le réacteur,
la membrane de fibres creuses a une limite de perméabilité qui est ajustée de telle façon qu'elle laisse passer les substances nutritives et les déchets des cellules, mais ne laisse pas passer les constituants protéiques de masse moléculaire élevée du milieu de culture,
à travers le volume intérieur de la membrane de fibres creuses, un milieu nutritif séparé du milieu de culture afflue à travers une conduite d'écoulement séparée du milieu de culture par la membrane de fibres creuses, et
le milieu de culture est en circulation dans le réacteur de manière à être amené au contact de la face extérieure de la membrane de fibres creuses,
de sorte que les substances nutritives passent, à travers les parois de la membrane de fibres creuses, à partir du milieu nutritif dans le milieu de culture, et les déchets passent du milieu de culture dans le milieu nutritif, mais les constituants protéiques de masse moléculaire élevée restent dans le milieu de culture.

2. Procédé selon la revendication 1, caractérisé en ce que la surface d'échange de la membrane de fibres creuses est d'au moins 0,01 m² par litre de milieu de culture et d'au plus 0,3 m² par litre de milieu de culture, de préférence d'au moins 0,03 m² par litre de milieu de culture et d'au plus 0,2 m² par litre de milieu de culture.

3. Dispositif pour la culture de cellules, comportant un réacteur (10), dans lequel un milieu de culture (12) est maintenu en suspension extrêmement homogène à l'aide d'un agitateur, et présente des conditions environnementales déterminées pour l'ajustement des dispositifs (52, 54, 46, 57), dans lequel
une membrane de fibres creuses (28) est prévue dans le réacteur, séparant une chambre de dialysat (34), dans laquelle est inclus le volume intérieur de la membrane de fibres creuses (28), du volume intérieur du réacteur (10),
la limite de perméabilité de la membrane de fibres creuses (28) est ajustée de telle façon qu'elle laisse passer les substances nutritives et les déchets des cellules, mais ne laisse pas passer les constituants protéiques de masse moléculaire élevée du milieu de culture,
la chambre de dialysat (34) est reliée à un réservoir de milieu nutritif (44) par l'intermédiaire d'une conduite d'amenée (48) et d'une conduite de retour (50), et il est fourni des dispositifs destinés à amener le milieu nutritif (44) du réservoir de milieu nutritif, par l'intermédiaire de la conduite d'amenée (48), dans la chambre de dialysat (34), et à le ramener de la chambre de dialysat (34), par l'intermédiaire de la conduite de retour (50), dans le réservoir, et
il est fourni un dispositif de recirculation au moyen duquel le milieu de culture (12) est en recirculation à l'intérieur du réacteur (10) de façon à être en contact avec la membrane de fibres creuses (28).

4. Dispositif selon la revendication 3, dans lequel la surface d'échange de la membrane de fibres creuses est d'au moins 0,01 m² par litre de milieu de culture et d'au plus 0,3 m² par litre de milieu de culture, de préférence d'au moins 0,03 m² par litre de milieu de culture et d'au plus 0,2 m² par titre de milieu de culture.

5. Dispositif selon l'une quelconque des revendications 3 ou 4, dans lequel le dispositif de recirculation comprend au moins un agitateur à ailettes (20).
